# EUROPÄISCHE PATENTSCHRIFT

(11) **EP 0 675 865 B1**
(45) Veröffentlichungstag und Bekanntmachung des Hinweises auf die Patenterteilung: **24.09.1997**
(21) Anmeldenummer: 94902732.0
(22) Anmeldetag: 09.12.1993
(51) Int. Cl.: C07C 23/08, C07C 23/06, C07C 23/10, C07C 17/26, C08F 14/18

(54) **METHYLENPERFLUORCYCLOALKANE UND DEREN VERWENDUNG ZUR HERSTELLUNG THERMOPLASTISCHER FLUORHARZE**
METHYLENEPERFLUOROCYCLOALKANES AND THEIR USE IN THE PRODUCTION OF THERMOPLASTIC FLUORORESINS
METHYLENEPERFLUOROCYCLOALCANES ET LEUR UTILISATION DANS LA FABRICATION DE RESINES FLUOREES THERMOPLASTIQUES

(30) Priorität: 22.12.1992 DE 4243526
(43) Veröffentlichungstag der Anmeldung: 11.10.1995
(73) Patentinhaber: BAYER AG, 51368 Leverkusen (DE)
(72) Erfinder: KRÜGER, Ralf, D-51061 Köln (DE); NEGELE, Michael, D-42697 Solingen (DE); LUI, Norbert, D-51060 Köln (DE); MARHOLD, Albrecht, D-51373 Leverkusen (DE)
(86) Internationale Anmeldenummer: EP9303470
(87) Internationale Veröffentlichungsnummer: WO9414738

(56) Entgegenhaltungen:
- GB-A- 1 171 065
- US-A- 3 274 265
- US-A- 3 894 097

## Beschreibung

Es ist bekannt, daß Hexafluoraceton (US-A 3 894 097) oder Hexafluoraceton-Hydrat (DE-A 3 425 907) mit Diketen. Acetanhydrid, Essigsäure oder Aceton bei Temperaturen von 340 bis 1 000°C über die Zwischenstufe Bis-(trifluormethyl)-β-propiolacton (I. L. Kumyats et. al., Izvest. Akad. Nauk SSR. 640 (1960) engl.) Hexafluorisobutylen (HFiB) bildet.

Es war überraschend und nach dem Stand der Technik nicht vorherzusehen, daß auch die Umsetzung von Keten-erzeugenden Verbindungen wie Diketen. Acetanhydrid, Essigsäure oder Aceton bei Temperaturen von 340 bis 1 000°C mit Perfluorcycloketonen zu Methylenperfluorcycloalkanen trotz des sterischen Anspruchs des intermediär gebildeten Spirolactons (J. March, Advanced Organic Chemistry, McGraw-Hill 1977. Kap. 4, S. 144f.) der Formel III mit befriedigenden Ausbeuten möglich ist.

Gegenüber dem "HFiB-Verfahren" erhält man erfindungsgemäß die Methylenperfluorocycloalkane isomerenrein. Aufgrund der Siedepunkte sind die Ausgangskomponenten und Nebenprodukte leicht abzutrennen (besonders ab n=2).

Fluorpolymere werden in der Technik immer dann eingesetzt, wenn besondere Eigenschaften wie niedrige Oberflächenspannung, hohe Chemikalien-, Öl- und Lösungsmittelbeständigkeit oder extreme Anforderungen an die (Hitze-)Alterungsstabilität gepaart mit hoher Wärmeformbeständigkeit verlangt werden.

Als größter Massenkunststoff auf dem Gebiet der Fluorpolymere vereinigt das Polytetrafluorethylen (PTFE) die genannten Eigenschaften in umfassenster Weise. Jedoch läßt sich PTFE bekanntermaßen thermoplastisch nicht verarbeiten. Wärmeformbeständigkeit und Gasdurchlässigkeit nehmen bei Temperaturen oberhalb 100°C rapide ab. Eine Verbesserung der thermoplastischen Verarbeitbarkeit erreicht man durch die Einführung von Comonomeren, die die Viskosität des Polymeren oberhalb des Erweichungspunktes (Schmelzpunkt bei teilkristallinen Systemen) herabsetzen und damit den Schmelzfluß verbessern. Solche Comonomeren sind beispielsweise Hexafluorpropen und perfluorierte acyclische Alkyl-vinylether. Meist sinkt aber mit dieser Maßnahme der Erweichungspunkt des Copolymeren. so daß man sich je nach Anwendungszweck mit Kompromißlösungen zufrieden geben muß.

Andere fluorhaltige Homopolymerisate wie das Polyvinylidenfluorid oder das Polychlortrifluorethylen sind zwar thermoplastisch verarbeitbar, erreichen aber wegen ihres geringeren Fluorgehaltes bei den o.g. Eigenschaften nicht das Niveau, das von höchstfluorierten (Co-)Polymeren erreicht wird. Durch Copolymerisation kann man auch hier entscheidende Verbesserungen erzielen.

Nach einem nicht vorveröffentlichten Vorschlag der Anmelderin zeigen Copolymere von Perfluor-(cycloalkyl-vinylethern) mit VDF oder CTFE verbesserte Thermostabilitäten im Vergleich zu den Homopolymeren.

Mit den in US-A 3 706 723 beschriebenen Copolymeren aus VDF und Hexafluorisobutylen (HFiB) werden höhere Schmelztemperaturen (>=300°C) als mit reinem PVDF (160 bis 170°C) erreicht. Wegen der schon bei 360°C beginnenden Zersetzung bieten solche Copolymeren allerdings nur ein enges Verarbeitungsfenster (TOMMASI, G.: Fluoropolymers Conference 1992. Manchester). Bei der HFiB-Synthese treten zudem hochtoxische Zwischenstufen auf. Copolymere des HFiB mit Vinylacetat (Vac) bzw. Vinylalkohol (VOH) (US-A 5 053 470) sind amorph und zeigen Glasübergänge bei ca. 45 bzw. 90°C. Sie sind jedoch wegen ihrer niedrigen Glastemperaturen, die ein Maß für die thermoplastische Erweichung darstellen, für viele Anwendungen untauglich.

Gegenstand dieser Erfindung sind neue Fluormonomerbausteine der Formel (1) 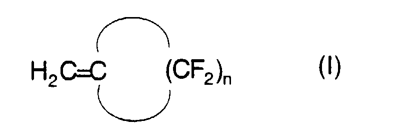 mit n = 3 - 5

aus denen eine Vielzahl thermoplastisch verarbeitbarer Copolymerisate mit unterschiedlichen Fluorgehalten hergestellt werden können, die sich durch hohe Wärmeformbeständigkeit Thermostabilität sowie Chemikalienbeständigkeit auszeichnen und je nach Comonomer unterschiedliche adhesive Eigenschaften und Löslichkeiten in organischen Solventien besitzen. Solche Copolymeren eignen sich je nach Comonomerzusammensetzung und dem daraus folgenden Eigenschaftsprofil sowohl für den Einsatz auf dem Gebiet thermoplastischer Formkörper als auch für Beschichtungen.

Methylentluoralkane der Formel (I) sind neu. Das Methylenfluoralkan der Formel (I) mit n = 4 ist nach Kenntnis der Anmelderin lediglich bereits einmal als hypothetische, intermediäre Struktur bei einem Versuch der Erklärung der bei der Bestrahlung von Decafluorcyclohexan mit UV-Licht entstehenden Endprodukte beschrieben (siehe Literaturstelle).

Erstmals werden erfindungsgemäß Methylenfluoralkane tatsächlich als stabile Endprodukte mit weniger als 30 Gew.-%, vorzugsweise weniger als 10 Gew.-% und besonders bevorzugt weniger als 5 Gew.-%, Verunreinigung durch Fremdprodukt zur Verfügung gestellt.

Die Methylenperfluorcycloalkane der Formel (I) wurden aus Perfluorcycloketonen der Formel (II) durch Umsetzung von Keten-erzeugenden Verbindungen Diketen, Acetanhydrid. Essigsäure oder Aceton bei Temperaturen von 340 bis 1 000°C hergestellt.

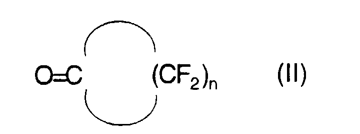

Die Keten-erzeugenden Verbindungen. z.B. Diketen. können beispielsweise in Mengen von 1 bis 5 Mol. bezogen auf 1 Mol Perfluorcycloketon der Formel (II) verwendet werden.

Bevorzugte Reaktionstemperaturen liegen im Bereich von 400 bis 700°C.

Die Verbindung der Formel (II) kann man nur mit der Keten-erzeugenden Verbindung oder im Gemisch mit inerten Gasen, z.B. Stickstoff, in der Gasphase umsetzen.

Die Reaktion kann man z.B. so durchführen, daß man Perfluorcycloketone der Formel (II) und Keten-erzeugende Verbindungen in einem oder mehreren parallel angeordneten Rohren aus inertem Material zuführt und das Rohr bzw. die Rohre auf die gewünschte Reaktionstemperatur erhitzt. Ein geeignetes Rohrmaterial ist z.B. Quarz.

Das Rohr bzw. die Rohre können gegebenenfalls mit inerten stückigen Materialien gefüllt sein, z.B. mit regelmäßig oder unregelmäßig geformten Quarzstücken mit einem mittleren Durchmesser von 1 mm bis zur Hälfte des inneren Durchmessers des jeweiligen Rohres.

Das aus der Reaktionszone kommende Gasgemisch kann man beispielsweise aufarbeiten, indem man es ganz oder teilweise kondensiert und aus dem Kondensat durch Destillation die darin enthaltenen Methylenperfluorcycloalkane isoliert.

Die als Nebenprodukte auftretenden Spirolactone der Formel (III) können ebenfalls durch eine Pyrolyse bei Temperaturen von 400 bis 700°C, bevorzugt 500 bis 600°C, in die gewünschten Methylenperfluorcycloalkane der Formel (I) überführt werden.

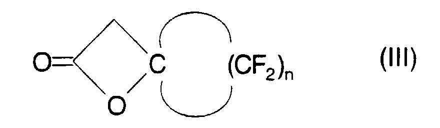

Es wurde gefunden, daß Methylen-perfluorcycloalkane der Formel (I) mit bestimmten fluorierten Monomeren wie Vinylidentluorid, Vinylfluorid und nichtfluorierten Monomeren wie Ethylen oder Vinylestern von kurzkettigen Carbonsäuren wie beispielsweise Vinylacetat, Vinylpropionat, Vinylbutyrat oder Vinylpivalat in guten Ausbeuten copolymerisieren.

Copolymere aus Methylen-perfluorcyclopentan und Vinylidenfluorid geben beispielsweise hochschmelzende Polymere (Tₘ bis 310°C) mit ausgezeichneter Temperatur- und Wärmeformbeständigkeit. Gegenüber perfluorierten Thermoplasten, die bezüglich ihrer Thermostabilität sowie Chemikalienbeständigkeit mit den erfindungsgemäßen Copolymeren vergleichbar sind, weisen die erfindungsgemäßen Copolymeren höhere Wärmeformbeständigkeiten bei geringeren Fluorgehalten auf.

Durch Copolymerisation der erfindungsgemäßen Monomeren mit Vinylestern wie beispielsweise Vinylacetat oder Vinylpropionat werden amorphe Copolymere erhalten, die durch Hydrolyse teilweise oder vollständig zu den Vinylalkohol-copolymeren überführt werden können. Solche Copolymeren aus Methylen-perfluorcycloalkanen und Vinylestern sowie die entsprechenden teilweise oder vollständig solvolysierten Produkte zeigen Glasübergänge bei über 100°C, sind in bestimmten organischen Lösungsmitteln löslich und eignen sich daher als Beschichtungsmaterialien mit guten Verarbeitungs- und Haftungseigenschaften sowie der Möglichkeit zur Vernetzung einerseits und den für Fluorpolymere typischen oben erwähnten Eigenschaften andererseits.

Gegenstand der vorliegenden Erfindung sind auch Polymerzusammensetzungen, die erhältlich sind durch Co-Polymerisation von
a) 10 bis 52 Mol-% Methylen-pertluorcycloalkan der Formel (1) und
b) 90 bis 48 Mol-% damit copolymerisierbaren Monomere, die entweder fluorhaltig sein können, wie
   b₁) Vinylidentluorid. Vinylfluorid, oder fluorfrei sein können, wie
   b₂) Ethylen. Vinylacetat oder Vinylalkohol (durch Verseifung von Vac-Co-polymeren).

Die Herstellung der erfindungsgemäßen Polymerisate erfolgt auf radikalischem Wege. Ansonsten gibt es keinerlei Beschränkung hinsichtlich der Polymerisationsmethode. Es kann in Substanz, Lösung (geeignete Lösungsmittel sind Fluorkohlen(wasser)stoffe. z.B. Hexafluorcyclopentan, Perfluorbutan oder Fluorchlorkohlenstoffe, z.B. Trichlortritluorethan), Suspension (unter Mitverwendung von Suspensionsstabilisatoren) oder Emulsion (fluorierte Emulgatoren sind notwendig) polymerisiert werden.

Der Radikalstart kann durch energiereiche Strahlung, thermisch oder durch Radikalinitiatoren eingeleitet werden. Zur chemischen Initiierung werden grundsätzlich bekannte und für das jeweilige Reaktionsmedium geeignete Verbindungen eingesetzt.

So verwendet man bei der Substanz-, Lösungs- oder Suspensionspolymerisation organische, öllösliche Peroxide, die auch fluoriert sein können, wie Diisopropylperoxydicarbonat, Trifluoracetylperoxid oder organische lösliche Azoverbindungen. wie Azobisisobutyronitril. Bei der Emulsionspolymerisation werden wasserlösliche anorganische Perverbindungen als Initiatoren benutzt. wie Persulfate, Perborate, Percarbonate etc., im allgemeinen in Form ihrer Kalium-, Natrium- oder Ammoniumsalze und bei Anwendung niederer Temperaturen gegebenenfalls in Kombination mit bekannten Zerfallsbeschleunigern, in der Regel Reduktionsmittel. Als solche können dienen: Schwefelverbindungen, wie etwa Natriumsulfit, Natriumpyrosulfit oder Rongalit C (Natriumformamidinsulfinsäure), weiterhin organische Reduktionsmittel. wie Ascorbinsäure, Metallsalze. wie Eisen-(II)- oder Kobalt-(II)-salze, metallorganische Verbindungen etc.

Die Polymerisationstemperaturen für die Copolymerisation liegen zwischen -30 und 90°C, bevorzugt nicht höher als 70°C.

Die Copolymerisation mit gasförmigen Monomeren wird unter erhöhtem Druck durchgeführt. Dieser Druck soll mindestens 2 bar betragen, braucht aber den Wert von 100 bar nicht zu überschreiten.

Es werden lineare Copolymere mit Molekulargewichten von 10³ bis 10⁶ g/mol erhalten.

### Beispiele

### Beispiel 1

### 3,3,4,4,5,5,6,6-Octafluormethylencyclopentan und (1-Hydroxyperfluorcyclopentyl)-essigsäure-β-lacton

In einem 30 cm langen (Durchmesser ca. 18 mm) Glasreaktor mit elektrischer Heizwicklung werden bei 500°C (± 10°C) aus zwei Tropftrichtern innerhalb von drei Stunden 114 g Octafluorcyclopentanon (0.5 mol) und 70 g frisch destilliertes Diketen (0.833 mol) unter einem leichten N₂-Strom eingefahren. Dabei werden die zudosierten Volumina so aufeinander abgestimmt, daß stets ein leichter Keten-Überschuß im Reaktor gewährleistet bleibt. Das Reaktionsgas wird bei -78°C kondensiert und langsam auf Raumtemperatur gebracht. Dabei destillieren CO₂ und überschüssiges Keten ab, wobei ca. 5 g mitgerissenes Material in einer nachgeschalteten, eisgekühlten Falle aufgefangen werden können. Anschließend wird das Rohgemisch (132 g) bei Normaldruck über Brücke grobdestilliert.

(Sumpf max. 110°C, Kopftemperatur max. 80°C); es werden 68 g Destillat erhalten.

Die Grobdestillate werden über eine 40 cm-Füllkörper-Kolonne bei Normaldruck fraktioniert: Neben dem Vorlauf von Octafluorcyclopenten (Kp 26 bis 28°C 12 g) und einem Zwischenlauf (Kp 30 bis 62°C lt. GC 78% Zielprodukt) erhält man 47 g des Zielproduktes (Kp 64 bis 67°C lt.GC 98,5 %). Aus dem Sumpf der Grobdestillation und der Kolonnendestillation wird das (1-Hydroxyperfluorcyclopentyl)essigsäure-β-lacton (16,5 g lt. GC 85 %) gewonnen.

Spektroskopische Daten von 3,3,4,4,5,5,6,6-Octafluormethylencyclopentan:
- ¹H-NMR:: δ = 6.55 ppm (q, J_{H-F} ≈ 3Hz, =CH₂) (gg. int. TMS in CDCl₃).
- ¹⁹F-NMR:: δ = -112,4 ppm (t, J_{H-F} ≈ 3Hz, 2CF₂ neben =CH₂) und -135,4 ppm /t. 2CF₂) (gg. CFCl₃ in CDCl₃).
- ¹³C-NMR:: δ = 111,2 ppm (¹J_{C-F} ≈ 266Hz, 4 CF₂); 129,5 ppm (s,β-C [CH₂=] und 134,6 ppm (q.α-C [R_{f}-C=], t, ³J_{C-F} = 23 Hz) (gg. int. TMS in CDCl₃).

Spektroskopische Daten von (1-Hydroxyperfluorcyclopentyl)essigsäure-β-lacton:
- ¹H-NMR:: δ = 3.97 ppm (s, CH₂) (gg. int. TMS in CDCl₃).
- ¹⁹F-NMR:: δ = -136,8 ppm (quar. 2CF₂), -141.3 ppm (quar, 2CF₂) (gg. CFCl₃ in CDCl₃).

### Beispiel 2

In einem 40 cm langen (Durchmesser ca. 25 mm) Quarzreaktor gefüllt mit Quarzbruch werden bei 550°C innerhalb von 1 Stunde 40g (1-Hydroxyperfluorcyclopentyl)essigsäure-β-lacton unter leichtem Stickstoffstrom eingefahren. Das Reaktionsgas wird in einem Kühler kondensiert und anschließend destilliert. Das Lacton wurde zu 100% umgesetzt. Ausbeute an Octafluormethylencyclopentan beträgt 70 %.

### Beispiel 3

Analog Beispiel 1 wurden 0,25 mol entsprechend 44,5 g Hexafluorcyclobutanon [gemäß J. Am. Chem. Soc. 83, 225 (1961)] und 35 g Diketen umgesetzt.

Es wurden 66 g Kondensat isoliert, aus denen nach Rektifikation bei Normaldruck 22 g 3,3,4,4,5,5-Hexafluormethylencyclobutan mit einem Siedepunkt von 42 bis 44°C erhalten wurden (= 49,5 %).

### Beispiel 4

Analog Beispiel 1 wurden 0,5 mol entsprechend 139 g Dekafluorcyclohexanon [gemäß J. Org. Chem 33, 2692 (1968)] und 70 g Diketen umgesetzt.

Es wurden 195 g Kondensat isoliert, aus denen nach Rektifikation bei Normaldruck 86 g (= 62,3 %) 3,3,4,4,5,5,6,6,7,7-Hexafluormethylencyclobutan mit einem Siedepunkt von 108 bis 110°C erhalten wurden.

### Beispiel 5

### Copolymerisation von Vinylidenfluorid mit Methylen-perfluorcyclopentan

In einem 0.3 1-Autoklaven wurden unter Rühren 100 g 1.1.2.2.3.3-Hexafluorcyclopentan, 60 mg Diisopropylperoxydicarbonat und 30 g Methylen-perfluorcyclopentan gegeben und auf -6°C gekühlt. Dann wurde der geschlossene Autoklav dreimal mit einem Stickstoffdruck von 10 bar beaufschlagt und jeweils anschließend auf Normaldruck entspannt. Danach wurden 20 g Vinylidentluorid in den Autoklaven einkondensiert. Das Reaktionsgemisch wurde unter konstanter Rührung auf 40°C erwärmt. Nach einer Gesamtreaktionszeit von 40 h bei 40°C wurde das Gemisch abgekühlt. Aus dem Gemisch wurden durch vollständige Ausfällung mit Ethanol und Trocknung bei 60°C im Vakuum 15 g eines weißen pulverförmigen Polymeren isoliert.

Das Copolymere ist nicht löslich in Trichlor-trifluorethan (R113). Aceton, Dimethylformamid sowie in Dimethylacetamid.

Die durch Fluorelementaranalyse (F-Gehalt: 62,2 Gew.-%) bestimmte Copolymerzusammensetzung beträgt 86 : 13 (molares Verhältnis VDF/Methylen-perfluorcyclopentan).

Dieses Copolymer schmilzt bei 307°C (DSC, Schmelzenthalpie: 30 J/g). Polymerdichte: 2.19 g/cm³.

### Beispiel 6

In einem 0,3 l-Autoklaven wurden 130 ml entionisiertes Wasser vorgelegt. Darin wurden 0,6 g Lithiumperfluoroctylsulfonat und 0,8 g Kaliumperoxidisulfat gelöst. Mit Natriumhydroxid wurde diese Lösung auf einen pH-Wert von etwa 10 eingestellt. Dann wurde der geschlossene Autoklav dreimal jeweils mit einem Stickstoffdruck von 10 bar beaufschlagt und anschließend auf Normaldruck entspannt. In den Autoklaven wurden 18 g Methylenperfluorcyclopentan und 20 g Vinylidenfluorid gegeben und das Reaktionsgemisch unter Rühren auf 70°C erwärmt. Nach einer Gesamtreaktionszeit von 10 h bei 70°C wurde das Gemisch abgekühlt. Nach Ablauf dieser Zeit, in der der Reaktionsdruck von 31 bar auf 29 bar abnahm, wurde der Autoklaveninhalt abgekühlt und das nicht umgesetzte Gasgemisch abgelüftet. Die so erhaltene Emulsion wurde zur vollständigen Koagulation in 130 ml einer 4-%igen wäßrigen Magnesiumsulfatlösung gegossen. Das Produkt wurde mit Wasser gewaschen und getrocknet, wobei man 10 g eines Copolymeren (weißes Pulver) bestehend aus Einheiten von Vinylidenfluorid und Methylen-perfluorcyclopentan erhielt.

Das Copolymer ist in den unter Beispiel 5 angegebenen Lösungsmitteln ebenfalls nicht löslich.

Die durch Fluorelementaranalyse (F-Gehalt: 64,2 Gew.-%) bestimmte Copolymerzusammensetzung beträgt 70 : 30 (molares Verhältnis VDF/Methylen-perfluorcyclopentan).

Dieses Copolymer schmilzt bei 308°C (DSC, Schmelzenthalpie: 23,6 J/g).

### Beispiel 7

Analog der im Beispiel 6 beschriebenen Verfahrensweise wurden 28 g Methylenperfluorcyclopentan und 12 g Vinylidenfluorid copolymerisiert. Es wurden 5,1 g eines Copolymeren (weißes Pulver) bestehend aus Einheiten von Vinylidenfluorid und Methylen-perfluorcyclopentan erhalten.

Das Copolymer ist in den unter Beispiel 5 angegebenen Lösungsmitteln ebenfalls nicht löslich.

Die durch Fluorelementaranalyse (F-Gehalt: 63.8 Gew.-%) bestimmte Copolymerzusammensetzung beträgt 73 : 27 (molares Verhältnis VDF/Methylen-perfluorcyclopentan). Dieses Copolymer schmilzt bei 309°C (DSC. Schmelzenthalpie: 30,6 J/g).

### Beispiel 8 (Vergleichsbeispiel)

Analog der im Beispiel 6 beschriebenen Verfahrensweise wurden 34,2 g Hexafluorisobutylen und 20 g Vinylidenfluorid copolymerisiert. Es wurden 9 g eines Copolymeren (weißes Pulver) bestehend aus Einheiten von Vinylidenfluorid und Hexafluorisobutylen erhalten.

Das Copolymer ist in den.unter Beispiel 5 angegebenen Lösungsmitteln ebenfalls nicht löslich.

Die durch Fluorelementaranalyse (F-Gehalt: 64,5 Gew.-%) bestimmte Copolymerzusammensetzung beträgt 72 : 28 (molares Verhältnis VDF/HFiB).

Dieses Copolymer schmilzt bei 303°C (DSC. breit verschmierter Schmelzpeak, Schmelzenthalpie: 5,7 J/g).

Es wurde versucht, die Polymerisate aus den Beispielen 5, 7 und 8 bei 340°C in einem Schmelztiegel unter Luft aufzuschmelzen. Dabei wurden folgende Beobachtungen gemacht:

| Beispiele | Zeitpunkt | | Beobachtungen |
|---|---|---|---|
| 5 | 1 min | → | farblos, geschmolzen, dünnflüssig |
| | 25 min | → | leichte Gelbfärbung, dünnflüssige Konsistenz der Schmelze bleibt erhalten |
| | | | |
| 7 | 0,5 min | → | leichte Gelbfärbung |
| | 1 min | → | geschmolzen, gelb |
| | | | |
| 8 | 0,5 min | → | braun |
| (Vergleich) | 1 min | → | geschmolzen, zähflüssig, tiefbraun |

### Beispiel 9

### Copolymerisation von Vinylacetat mit Methylen-perfluorcyclopentan

In einem 300 ml Glaskolben wurden 100 g 1,1,2,2,3,3-Hexafluorcyclopentan vorgelegt und nach Kühlung auf -50°C 36,2 g Methylenperfluorcyclopentan und 13,8 g Vinylacetat sowie 0,3 g Diisopropylperoxydicarbonat unter Rühren zugegeben. Die Reaktionsapparatur wurde dann 3 mal bis auf etwa 4 mbar evakuiert und jeweils mit Stickstoff belüftet. Das Reaktionsgemisch wurde unter konstanter Rührung auf 30°C erwärmt. Während der Umsetzung wurde der Feststoffgehalt der Lösung kontrolliert. Er betrug nach 24 h 13,1 % und nach 45 h 24 %. Nach einer Gesamtreaktion von 48 h bei 30°C wurde das Gemisch abgekühlt. Es wurde eine viskose farblose Lösung mit einem Feststoffgehalt von 25,2 Gew.-% erhalten und durch Einrühren in Ethanol gefällt. Dadurch wurden 35 g eines weißen pulverförmigen Polymeren isoliert.

Das Polymer ist löslich in Tetrahydrofuran und 1,1,2-Trichlortrifluorethylen (R113) sowie unlöslich in Aceton, Chloroform, Dimethylfomamid und Dimethylacetamid. Der Staudingerindex [η] (auch inhärente Viskosität) in THF beträgt 0,1 dl/g.

Die chemische Zusammensetzung wurde durch ¹H-Kernresonanzspektroskopie (200 MHz in THF) bestimmt (Auswertung der Signale bei 5,7 ppm für CH sowie bei 2....2,6 ppm für CH₂ und CH₃). Das molare Verhältnis von Vinylacetat zu Methylen-perfluorcyclopentan beträgt danach 51 : 49 nach 24 h Polymerisationszeit und 47 : 53 nach Beendigung der Polymerisation (48 h). Im IR-Spektrum beobachtet man bei 1 679 cm⁻¹ eine intensive Bande, die von der Carbonylschwingung des Acetatrestes verursacht wird.

Durch DSC-Analyse wurde eine Glastemperatur bei 92°C (2. Aufheizung) gefunden. Schmelzbereiche wurden nicht beobachtet. An einer Preßplatte (4 x 4 x 1 mm) wurde eine Thermomechanische Analyse durchgeführt. Das Material zeigt eine typische Thermoplasterweichung, die im Bereich der Glastemperatur (90 bis 100°C) beginnt und bei ca. 120°C endet.

### Beispiel 10

### Verseifung von Vinylacetat/Methylen-perfluorcyclopentan-Copolymeren

5 g des nach Beispiel 9 hergestellten Copolymeren aus Vinylacetat und Methylenperfluorcyclopentan wurden in 50 ml Tetrahydrofuran gelöst und unter Rühren langsam 33 ml einer Suspension aus THF und 2,25 g Kaliumhydroxid (2,5 facher molarer Überschuß, bezogen auf Acetoxygruppen). Nach der exothermen Reaktion (Temperaturerhöhung von 22 auf 24°C) wurde die Lösung 3 h bei 50°C gerührt. Danach wurde das Polymere durch Einrühren in Wasser, das mit Essigsäure auf pH 1,8 angesäuert wurde, gefällt, getrocknet und zweimal aus THF/H₂O umgefällt. Es werden 2,8 g eines weißen bis schwach beigen Pulvers erhalten.

Löslichkeit:
Tetrahydrofuran (gut)
1,1,2-Trichlor-trifluorethan (gut)
Aceton (teilweise)
1,1,1-Trichlorethan (teilweise)
1,1,2,2,3,3-Hexafluor-cyclopentan (teilweise)

Der Staudingerindex in THF beträgt 0.09 dl/g, woran zu erkennen ist, daß kein Abbau stattgefunden hat, da das Ausgangspolymer mit 0,1 dl/g (Beispiel 9) etwa den gleichen [η] Wert zeigt (die geringe Abnahme ist auf die Gewichtsabnahme durch die Acetatabspaltung zurückzuführen).

Im IR-Spektrum beobachtet man eine Abnahme der relativen Intensität der Carbonylschwingungsbande bei 1 679 cm⁻¹ gegenüber der des nicht verseiften Ausgangspolymeren (Beispiel 9) um 20 %. Das nach diesem Beispiel hergestellte Terpolymere setzt sich somit aus ca. 9 Mol-% Vinylalkohol-, 38 Mol-% Vinylacetat- und 53 Mol-% Methylen-perfluorcyclopentan-Einheiten zusammen.

Auch diese Probe ist amorph und zeigt einen Glasübergang bei 125°C. Die Teilhydrolyse führt somit zu einer Erhöhung des Glasübergangs.

## Patentansprüche

1. Methylen-perfluorcycloalkane der Formel 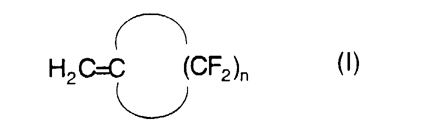 mit n = 3. 4 oder 5.

2. 3,3,4,4,5,5,6,6-Octafluormethylencyclopentan.

3. Verfahren zur Herstellung von Methylen-perfluorcycloalkanen der Formel (I) mit n = 3 bis 5, dadurch gekennzeichnet, daß keten-erzeugende Verbindungen mit Perfluorcycloketonen der Formel 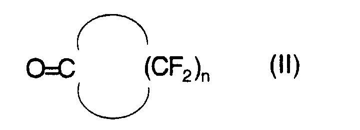 bei Temperaturen von 340 bis 1 000°C direkt miteinander oder im Gemisch mit inerten Gasen umgesetzt werden.

4. Verwendung von Methylen-perfluorcycloalkane nach Anspruch 1 oder 2 zur Herstellung von thermoplastischen Fluorharzen.

5. Polymerzusammensetzungen, erhältlich durch radikalische Copolymerisation von
a) 10 bis 52 Mol-% Methylen-perfluorcycloalkanen nach Anspruch 1 oder 2, und
b) 90 bis 48 Mol-% Vinylidenfluorid. Vinylfluorid, Ethylen, Vinylacetat und/oder Vinylalkohol (durch Verseifung von Vinylestercopolymeren).

## Claims

1. Methyleneperfluorocycloalkanes corresponding to the formula 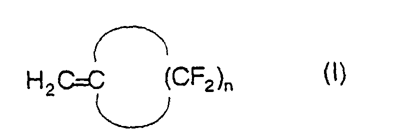 wherein n equals 3, 4 or 5.

2. 3,3,4,4,5,5,6,6-octafluoromethylenecyclopentane.

3. Method for the preparation of methyleneperfluorocycloalkanes of the formula (I), wherein n equals 3 to 5, characterised in that ketene-producing compounds are reacted with perfluorocycloketones corresponding to the formula 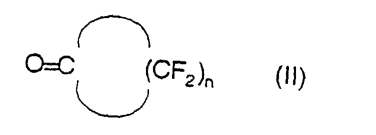 at temperatures of from 340 to 1000°C, directly with one another or mixed with inert gases.

4. Use of methyleneperfluorocycloalkanes according to claim 1 or 2 for the production of thermoplastic fluororesins.

5. Polymer compositions, obtainable by radical copolymerisation of
a) from 10 to 52 mol-% of methyleneperfluorocycloalkanes according to claim 1 or 2, and
b) from 90 to 48 mol% of vinylidene fluoride, vinyl fluoride, ethylene, vinyl acetate and/or vinyl alcohol (by saponification of vinyl ester copolymers).

## Revendications

1. Méthylèneperfluorocycloalcanes de formule 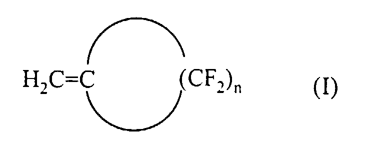 dans laquelle n = 3, 4, ou 5

2. Le 3,3,4,4,5,5,6,6-octafluorométhylènecyclopentane.

3. Procédé de préparation des méthylèneperfluorocycloalcanes de formule (I) dans laquelle n = 3 à 5, caractérisé en ce que l'on fait réagir des composés produisant du cétène avec des perfluorocyclocétones de formule 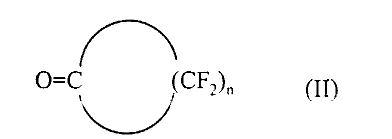 à des températures de 340 à 1 000°C, directement ou en mélange avec des gaz inertes

4. Utilisation des méthylèneperfluorocycloalcanes selon la revendication 1 ou 2 pour la préparation de résines fluorées thermoplastiques

5. Compositions polymères obtenues par copolymérisation radicalaire de
a) 10 à 52 mol % de méthylèneperfluorocycloalcanes selon revendication 1 ou 2 et
b) 90 à 48 mol % de fluorure de vinylidène, de fluorure de vinyle, d'éthylène, d'acétate de vinyle et/ou d'alcool vinylique (par saponification des copolymères d'esters vinyliques).
